# EUROPÄISCHE PATENTSCHRIFT

(11) **EP 0 778 823 B1**
(45) Veröffentlichungstag und Bekanntmachung des Hinweises auf die Patenterteilung: **02.06.1999**
(21) Anmeldenummer: 95930490.8
(22) Anmeldetag: 17.08.1995
(51) Int. Cl.: C07C 213/00, C07D 263/24

(54) **VERFAHREN ZUR REDUKTION VON AMINOSÄUREN UND DEREN DERIVATEN**
PROCESS FOR REDUCING AMINO ACIDS AND DERIVATIVES THEREOF
PROCEDE DE REDUCTION DES AMINOACIDES ET DE LEURS DERIVES

(30) Priorität: 03.09.1994 DE 4431529
(43) Veröffentlichungstag der Anmeldung: 18.06.1997
(73) Patentinhaber: Degussa Aktiengesellschaft, 60311 Frankfurt (DE); F. HOFFMANN-LA ROCHE AG, 4002 Basel (CH)
(72) Erfinder: KOTTENHAHN, Matthias, D-63579 Freigericht (DE); DRAUZ, Karlheinz, D-63579 Freigericht (DE); HILPERT, Hans, CH-4153 Reinach (CH)
(86) Internationale Anmeldenummer: EP9503281
(87) Internationale Veröffentlichungsnummer: WO9607634

(56) Entgegenhaltungen:
- EP-A- 0 326 934
- DE-A- 4 232 505
- BULL. CHEM. SOC. JPN. (BCSJA8,00092673);84; VOL.57 (8); PP.2327-8, SCI. UNIV. TOKYO;FAC. SCI.; TOKYO; 162; JAPAN (JP), SOAI K ET AL 'The preparation of N-protected amino alcohols and N-protected peptide alcohol by reduction of the corresponding esters with sodium borohydride. An improved procedure involving a slow addition of a small amount of methanol' in der Anmeldung erwähnt
- CHEMICAL ABSTRACTS, vol. 101, no. 17, 22.Oktober 1984 Columbus, Ohio, US; abstract no. 152278, SOAI K ET AL 'Novel procedure of the functional group selective reduction of esters with sodium borohydride by a slow addition of methanol. Application to the reduction of N-protected amino acid ester' & PEPT. CHEM. (PECHDP);84; VOL.21ST.,; PP.85-8, SCI. UNIV. TOKYO;FAC. SCI.; TOKYO; 162; JAPAN (JP),

## Beschreibung

Die Erfindung betrifft ein Verfahren zur Reduktion von Aminosäuren oder Aminosäurederivaten der Formel I 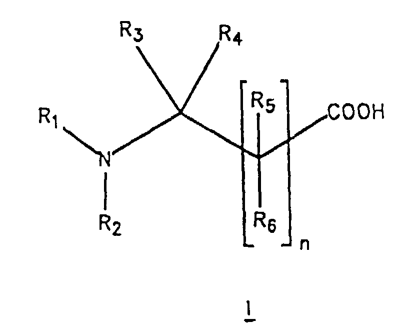 worin
- n =: 0 oder 1 ist,
- R¹, R², R³, R⁴, R⁵ und R⁶: unabhängig voneinander gleich oder verschieden H, Aryl, Alkyl oder Arylalkyl bedeuten, wobei bei den beiden letzteren die Kohlenstoffkette mit Heteroatomen wie N, O oder S oder solche Atome aufweisenden Gruppen substituiert und/oder unterbrochen sein kann,
außerdem
- R¹ und R²: auch noch unabhängig voneinander gleich oder verschieden Arylalkyloxycarbonyl, Alkyloxycarbonyl oder Oxycarbonyl bedeuten kann,
außerdem
- R³, R⁴, R⁵ und R⁶: auch noch unabhängig voneinander gleich oder verschieden Acylaminoalkyl oder Hydroxyl bedeuten können,
und
wobei
- R³, R⁴, R⁵ oder R⁶: mit R¹ oder R² einen Ring bilden kann, zu den entsprechenden Aminoalkoholen der Formel II 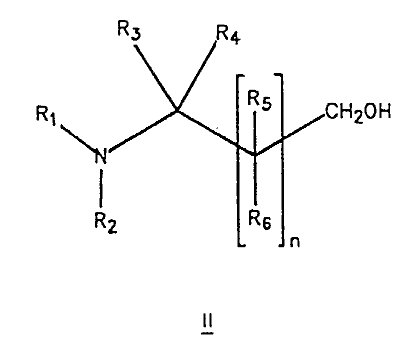
worin R¹ bis R⁶ und n die bei Formel I angegebene Bedeutung besitzen,
bei welchem Verfahren die Verbindungen der Formel I in einem ersten Schritt zunächst in wenigstens einem linearen oder verzweigtkettigen Alkohol mit 1 - 5 C-Atomen unter Zusatz von Säure und unter Erwärmen zu einem Ester umgesetzt werden, wobei eine den Ester enthaltende Reaktionsmischung resultiert und der Ester in einem zweiten Schritt mit Alkali- oder Erdalkaliborhydrid zu Verbindungen der Formel II reduziert wird.

Für den Fall, daß in Verbindungen vom Typ der Formel I, II einer der Reste R³, R⁴, R⁵ oder R⁶ mit einem der Reste R¹ oder R² einen Ring bildet, versteht es sich, daß sich entsprechend den Regeln der chemischen Nomenklatur die Bezeichnung der beiden an der Ringformulierung beteiligten Reste ändert. Wenn also der Fall der Ringbildung vorliegt, sind die entsprechenden Reste R¹ bis R⁶, die konkret an der Ausbildung des Rings teilhaben, entweder als Alkylen oder Arylalkylen, wobei deren Kohlenstoffkette oder -ring mit Heteroatomen wie N, O und/oder S oder solche Atome aufweisenden Gruppen substituiert und/oder durch Heteroatome wie N, O und/oder S unterbrochen sein kann, zu bezeichnen, R¹ und R² können aber außerdem auch Carbonyl, Arylalkylenoxycarbonyl, Alkylenoxycarbonyl oder Oxycarbonyl und R³, R⁴, R⁵ oder R⁶ Arylen, Acylaminoalkylen oder Sauerstoff sein.
Wie gesagt, diese Bezeichnungen sind nur für die konkret zum Ring zusammengeschlossenen Reste zu verwenden. Ansonsten gilt für die nicht am Ringschluß beteiligten Reste die allgemein angegebene Bezeichnung.

Die durch die Reduktion von Aminosäuren oder Aminosäurederivaten erhältlichen Aminoalkohole sind vielseitig verwendbare Synthesebausteine. Insbesondere entsprechende chirale Verbindungen sind wichtige Komponenten, zum Beispiel zur Darstellung optisch aktiver Verbindungen.

Eine Übersicht über Aminoalkohole geben G. M. Coppola und H. F. Schuster in: Asymmetric Synthesis, John Wiley and Sons, New York 1987; oder M. Nogradi, Stereoselctive Synthesis, Verlag Chemie, Weinheim 1987.

So dienen die Aminoalkohole zur Herstellung chiraler Katalysatoren, mit denen enantioselektive Cyclopropanierungen, Reduktionen, Hydrosilylierungen, Diels-Alder-Reaktionen und andere Reaktionen durchgeführt werden können. Eine Übersicht hierzu findet man bei C. Bolm, Angew. Chem. 1991, 103, 556. Häufig werden die Aminoalkohole in wichtige Synthesezwischenprodukte eingebaut, um bei Folgereaktionen aufgrund sterischer oder elektronischer Einflüsse die Bildung von Asymmetriezentren zu induzieren, wobei die erzielten Enantio- oder Diastereoselektivitäten häufig außerordentlich hoch sind. Als Beispiele seien 4-substituierte Oxazolidin-2-one (J. R. Gage, D. A. Evans, Org. Synth. 1989, 68, 77 und dort zit. Lit.) und die bicyclischen Lactame (D. Romo, A. I. Meyers, Tetrahedron 1991, 47, 9503) genannt.

Aminoalkohole können auch in Peptidisostere integriert werden, wobei Produkte mit hoher physiologischer Aktivität zugänglich sind, zum Beispiel Enkephalinanaloga mit stärkerer und länger anhaltender analgetischer Wirkung (Y. Kiso, M. Yamaguchi, T. Akita, H. Moritoki, M. Takei, H. Nakamura, Naturwissenschaften 1981, 68, 210; J. Pless, W. Bauer, F. Cardinaux, A. Closse, D. Hauser, R. Huguenin, D. Roemer, H. H. Buescher, R. C. Hill, Helv. Chim. Acta 1919, 62, 398) oder auch HIV-Protease-Inhibitoren (z. B. T. F. Tam, J. Carriere, I. D. Macdonald, A. L. Castehano, D. H. Pliura, N. J. Dewdney, E. M. Thomas, C. Bach, J. Barnett, H. Chan, A. Krantz, J. Med. Chem. 1992, 35 (7), 1318). Auch als C-terminale Schutzgruppe sind Aminoalkohole in der Peptidsynthese geeignet (C. Kashima, K. Harada, Y. Fujiioka, T. Maruyama, Y. Omote, J. Chem. Soc. Perkin Trans. I 1988, 535).

Zur Spaltung von Racematen wurden chirale Aminoalkohole eingesetzt, indem entweder diastereomere Salze von N-Alkylaminoalkoholen fraktioniert kristallisiert (M. Tukamoto, T. Sawayama, Dainippon Pharmaceutical Co., EP 0 105 696 A1, 18.04.1984, F. Horiuchi, M. Matsui, Agric. Biol. Chem. 1973, 37, 1713) oder diastereomere Additionsverbindungen chromatographisch getrennt wurden (K. Ogura, M. Ishida, H. Tomori, M. Fujita, Bull. Chem. Soc. Jpn. 1989, 62, 3531).

Bei von Aminoalkoholen abgeleiteten 1,3,2-Oxazaphospholidin-2-sulfiden schließlich wurde insektizide Aktivität nachgewiesen (S. Wu, R. Takeya, M. Eto, C. Tomizawa, J. Pesticide Sci. 1987, 12, 221).

Zur Darstellung optisch aktiver Aminoalkohole existieren verschiedene Verfahren. Sie können z. B. durch Racematspaltungen erhalten werden, die entweder als konventionelle fraktionierte Kristallisation diastereomerer Salze wie im Falle des (S)-2-Aminobutanols, der Vorstufe des Tuberkolosestatikums Ethambutol (F. Lanzendörfer, G. Fritz, H. Siegel, BASF AG, DE 35 17 108 A1, 13.11.1986 und dort zit. Lit.), oder durch enzymatische Spaltung geeigneter Derivate ausgeführt werden können (F. Francalani, P. Cesti, W. Cabri, D. Bianchi, T. Martinengo, M. Foa, J. Org. Chem. 1987, 52, 5079 und dort zit. Lit., H. S. Bevinakatti, R. V. Nevadklar, Tetrahedron: Asym. 1990, 1, 583).

Prinzipiell verschieden davon sind reduktive Verfahren. Optisch aktive Aminoalkohole wurden erstmals durch Reduktion optisch aktiver Aminosäureester erhalten, obgleich mit erheblicher Racemisierung (P. Karrer, W. Karrer, H. Thomann, E. Horlacher, W. Mäder, Helv. Chim. Acta 1921, 4, 76). Später konnte diese Reaktion auch unter Verwendung von Lithiumaluminiumhydrid (P. Karrer, P. Portmann, M. Suter, Helv. Chim. Acta 1948, 31, 1617; P. S. Verkateswaran, T. J. Bardo, J. Org. Chem. 1967, 32, 1256), Natriumborhydrid (H. Seki, K. Koga, H. Matsuo, S. Ohki, I. Matsuo, S. Yamada, Chem. Pharm. Bull. 1965, 13, 995), Lithiumborhydrid (Barton u. Ollis, Comprehensive Organic Chemistry, Band 3 (1979), Seite 773 unten) oder Natriumborhydrid mit Methanol als Aktivator (Bull. Chem. Soc. Jpn. 57 (1984), S. 2327f und Chem. Abstr. 101 (1984), 152278p) durchgeführt werden, wobei gezeigt werden konnte, daß diese und andere Hydridreduktionen praktisch ohne Racemisierung verlaufen (G. S. Poindexter, A. I. Meyers, Tetrahedron Lett. 1977, 3527). Allerdings ist bei der "Aktivierung" mit Methanol u. a. die Vermischung mit Lösungsmitteln nachteilig, weil sie einer großtechnischen Wiederaufarbeitung der Lösungsmittel entgegensteht. Wegen des zeitaufwendigen zweistufigen Verfahrens mit Zwischenisolierung des Esters und den hohen Hydridüberschüssen bei Verwendung von Alkaliborhydriden hat sich als Standardverfahren die Reduktion der freien optisch aktiven Aminosäuren mit Lithiumaluminiumhydrid etabliert (D. A. Dickmann, A. I. Meyers, G. A. Smith, R. E. Gawley, Organic Syntheses Coll. Vol. VII, S. 539, John Wiley & Sons, New York 1990). Ebenso kann ein Boran-Dimethylsulfid-Komplex mit Aktivierung durch Bortrifluorid-Etherat eingesetzt werden (J. R. Gage, D. A. Evans, Org. Synth. 1989, 68, 77; G. A. Smith, R. E. Gawley, Org. Synth. 1985, 63, 136).

In jüngster Zeit konnte gezeigt werden, daß bei geeigneter Aktivierung auch Lithiumborhydrid (mit Trimethylchlorsilan: R. Dharanipragada, A. Alarcon, V. J. Hruby, Org. Prep. Proc. Int. 1991, 23, 396; mit Bortrifluorid-Etherat: W. H. J. Boesten, C. H. M. Schepers, M. J. A. Roberts (Stamicarbon B. V.) EP 0 322 982 A2, 05.07.1989) oder die Kombination NaBH₄-H₂SO₄ (A. Abiko, S. Masamune, Tetrahedron Lett. 33(28), 1992, 5517) für die direkte Reduktion von Aminosäuren verwendet werden können.

Alle genannten Verfahren besitzen jedoch Nachteile, die einer Anwendung in größerem Maßstab bisher im Wege standen. So sind die zweistufigen Reaktionen über die isolierte Esterzwischenstufe von vornherein zu zeitaufwendig und durch die hohen Hydridüberschüsse zusätzlich teuer. Reduktionen mit Lithiumaluminiumhydrid sind im Labormaßstab zwar schnell und effizient durchführbar, erfordern technisch aufgrund der leichten Entzündlichkeit des Hydrides jedoch besondere Vorsichtsmaßnahmen. Außerdem ist das Reagens durch seinen hohen Preis unwirtschaftlich. Der Einsatz eines Boran-Dimethylsulfid-Adduktes ist mit einer beträchtlichen Geruchsbelästigung verbunden. Bei den Methoden, die Bortrifluorid-Etherat als Aktivator verwenden, wird dieses Reagens in molaren Mengen eingesetzt, wodurch zunehmend Probleme bei der Entsorgung der fluoridhaltigen Abfälle entstehen. Ein Zusatz von Alkylhalogensilanen zur Aktivierung des Alkaliborhydrids führt zur Bildung nicht weiter verwendbarer Silane oder Siloxane. Die Kombination NaBH₄-H₂SO₄ kann bisher nur in Ethern als Lösungsmittel durchgeführt werden, wobei diese entweder teuer oder sicherheitstechnisch bedenklich sind. Zudem müssen erhebliche molare Überschüsse NaBH₄ eingesetzt werden.
Aus Tetrahedron Lett. 33, 5517 - 8, 1992 und J. Org. Chem. 1993, 58, 3568 sind verschiedene Reduktionsverfahren basierend auf NaBH₄ für freie bzw. N-geschützte Aminosäuren bekannt, u. a. das System NaBH₄-I₂. Reduziert wird dabei ausschließlich die Carbonsäure, jedoch sind bei einigen Systemen trotz einfacher Aminosäuren (z. B. L-Valin) die Ausbeuten sehr gering und des weiteren ist der Anfall von Iodabfall im größeren Maßstab mit großem Entsorgungs- oder Rezyklierungsaufwand verbunden.

Angesichts der Nachteile der im Stand der Technik vorhandenen Verfahren ist es Aufgabe der Erfindung, ein Verfahren zur Reduktion von Aminosäuren oder deren Derivaten zur Darstellung der korrespondierenden Aminoalkohole aufzuzeigen, bei dem die Reduktion unter milden Bedingungen und unter Erhalt eventueller Chiralitätszentren durchführbar sein soll. Die Reaktion soll mit preiswerten und auch im technischen Maßstab leicht und sicher handhabbaren Reagentien durchführbar sein, wobei nach Ende der Reaktion eine leichte Aufarbeitung des Produkts und eine unproblematische Entsorgung der Reststoffe gewährleistet sein soll.

Erfindungsgemäß werden diese und weitere nicht näher genannten Aufgaben durch ein eingangs beschriebenes Verfahren mit den Merkmalen des kennzeichnenden Teils des Anspruchs 1 gelöst. Vorteilhafte Verfahrensmodifikationen werden in den abhängigen Ansprüchen unter Schutz gestellt.

Durch die Veresterung der Aminosäure oder des Aminosäurederivats und anschließende Reduktion mit nicht aktivierten Alkali- oder Erdalkaliborhydriden ohne Zwischenisolierung der Ester gelingt es, aus Aminosäuren oder Aminosäurederivaten die entsprechenden Alkohole in einem einfachen "Eintopf"-Verfahren in hoher Ausbeute unter Erhalt eines ggf. vorhandenen Chiralitätszentrums herzustellen.

Die Reduktion von α-Aminosäurederivaten zu den α-Aminoalkoholen über eine Aminosäureesterzwischenstufe mit Natriumborhydrid ist zwar grundsätzlich bekannt (H. Seki, K. Koga, H. Matsuo, S. Ohki, I. Matsua, S. Yamada, Chem. Pharm. Bull. 13 (8), 995, 1965; S. Mandal, B. Achari, S. Chattopadhyay Tetrahedron Lett. 33 (12), 1992, 1647), jedoch war dieses Verfahren durch die vorherige Isolierung und Trocknung der Ester bzw. deren Salze, eventuell nachfolgende Freisetzung aus den Salzen mit Lauge und nachfolgender Reduktion mit 4 - 5 Molequivalenten NaBH₄ aufwendig, zeitintensiv und teuer.

Im Gegensatz hierzu vermeidet das Verfahren der Erfindung die nachteilige Isolierung des Zwischenprodukts.

Das erfindungsgemäße Verfahren kann in zwei theoretische Schritte oder Stufen unterteilt werden. Die erste Stufe umfaßt dabei die Veresterung der Aminosäuren oder der Aminosäurederivate, während die zweite Stufe die eigentliche Reduktion der Esterzwischenprodukte beinhaltet. Es ist jedoch erfindungswesentlich und wird ausdrücklich festgestellt, daß in der Praxis der Erfindung die beiden Stufen oder Schritte ohne Isolierung des Produkts der ersten Reaktionsstufe, also des Zwischenprodukts aus der resultierenden Reaktionsmischung, nacheinander ausgeführt werden.

Es wurde nun gefunden, daß trotz des Verzichts auf eine Isolierung der als Zwischenprodukt des erfindungsgemäßen Verfahrens am Ende des ersten Reaktionsschrittes auftretenden Ester die gesamte Reaktion zu einer hohen Ausbeute geführt werden kann, wenn zur Reduktion des Zwischenprodukts lediglich Alkali- oder Erdalkaliborhydrid ohne Zusatz einer die Reduktionsmittel aktivierenden Substanz zur aus dem ersten Schritt resultierenden Reaktionsmischung zugesetzt wird. Dieses Ergebnis war nicht zu erwarten, da bislang immer die Verwendung "sauberer" Zwischenprodukte oder die Aktivierung des Borhydrids für eine befriedigende Ausbeute zwingend erschien.

Die Veresterung als erster Schritt des erfindungsgemäßen Verfahrens wird nach literaturbekannten Verfahren (Houben Weyl, Bd. 15) durch Suspendieren oder Lösen der zu reduzierenden Aminosäure oder des entsprechenden Aminosäurederivats in einem C₁-C₅-Alkohol unter Zusatz von Säure und Erwärmen der Reaktionslösung bis eventuell zum Siedepunkt der Reaktionsmischung durchgeführt.

Zu den mit Erfolg im Rahmen der Erfindung einsetzbaren Alkoholen gehören beispielsweise in einer nicht abschließenden Aufzählung lineare Alkohole wie Methanol, Ethanol, Propanol, Butanol und verzweigtkettige Alkohole wie Isopropanol. Bevorzugt werden Methanol und Ethanol als Alkohole eingesetzt.

Säuren, die mit Erfolg zugesetzt werden, sind beispielsweise HCl, H₂SO₄, 4-Toluolsulfonsäure, Methansulfonsäure, Benzolsulfonsäure, Polysiloxansulfonsäuren sowie Mischungen von zwei oder mehreren der vorgenannten Komponenten. Unter den aufgezählten Säuren sind HCl, H₂SO₄ oder p-TsOH oder Mischungen davon bevorzugt.

Die Säuren oder Säuremischungen werden im allgemeinen in einer Menge von 0,05 - 3 moleq, bevorzugt 0,1 - 1,5 moleq, bezogen auf die zu reduzierende Aminosäure oder das zu reduzierende Aminosäurederivat, zugesetzt.

Handelt es sich bei der zu reduzierenden Aminosäure oder bei dem zu reduzierenden Aminosäurederivat um eine am Stickstoff der Aminogruppe nicht geschützte Aminosäure, wird zusätzlich zu den für die Veresterungskatalyse notwendigen Säuremengen ein Equivalent Säure zur Neutralisation der Aminofunktion benötigt.

Nach Beendigung des ersten oder Veresterungsschrittes kann gemäß der Erfindung grundsätzlich direkt mit dem zweiten oder Reduktionsschritt, d. h. der Reduktion des intermediär gebildeten Esterproduktes, fortgefahren werden. Es ist im Rahmen der Erfindung allerdings auch möglich und bevorzugt, das Zwischenprodukt des ersten Schrittes, das in Form einer Lösung vorliegen kann, ggf. aufzukonzentrieren, indem überschüssige flüssige Bestandteile der Reaktionsmischung entfernt werden, beispielsweise durch Abdampfen oder Abdestillieren. Dieses Aufkonzentrieren dient insbesondere dazu, die Veresterung zu komplettieren, Lösungsmittel zurückzugewinnen und die Volumina bei dem Reduktionsschritt zu begrenzen.

Des weiteren ist es ebenfalls möglich, in der Lösung des Zwischenprodukts vorhandenen Säureüberschuß ggf. durch Zugabe von Basen zu neutralisieren. Hierfür sind grundsätzlich alle dem Fachmann geläufigen basischen Substanzen verwendbar, bevorzugt werden jedoch Alkalien wie beispielsweise Natriumhydroxid, Natronlauge, Kaliumhydroxid, Kalilauge, Natriumalkoholat, Kaliumalkoholat, Natriumcarbonat, Natriumhydrogencarbonat, Kaliumcarbonat oder Kaliumhydrogencarbonat eingesetzt. Durch diese Maßnahme kann es insbesondere vorteilhaft gelingen, den zu verwendenden Hydridüberschuß in der sich anschließenden Reduktionsstufe ggf. noch weiter zu verringern.

Zu der wie geschildert ggf. optionell behandelten Reaktionsmischung,die nach dem ersten Schritt oder Veresterungsschritt resultiert, werden dann im Rahmen des zweiten Reaktionsschrittes der Erfindung 1 - <5 Molequivalente Alkali- oder Erdalkaliborhydrid als Reduktionsmittel zugesetzt.

Zu den mit Erfolg zu verwendenden Reduktionsmitteln gehören erfindungsgemäß u. a. NaBH₄, LiBH₄, KBH₄, Ca(BH₄)₂. Besonders bevorzugte Alkali- oder Erdalkaliborhydride sind: NaBH₄ oder LiBH₄.

Angesichts der bislang herrschenden Meinung ist es als durchaus unerwartet zu bezeichnen, daß in bevorzugter erfindungsgemäßer Verfahrensmodifikation die Menge des zur Reduktion hinzuzufügenden Alkali- oder Erdalkaliborhydrids deutlich unter den bislang zu verwendenden, auf den zur reduzierenden Ester bezogenen, ca. 5fachen molaren Überschuß gesenkt werden konnte, ohne dabei Ausbeuteverluste hinnehmen zu müssen.

In weiterhin bevorzugter Ausführungsform wird das Reduktionsmittel lediglich in 1,1 bis 3fachem molaren Überschuß, bezogen auf den als Zwischenprodukt resultierenden Ester, verwendet. Besonders bevorzugt kommt nur eine zwischen dem 1,2 und 2fachen molaren Überschuß liegende Menge des Alkali- oder Erdalkaliborhydrids zum Einsatz.

Die Temperatur für die Durchführung des eigentlichen Reduktionsschrittes ist grundsätzlich nicht kritisch, sie sollte jedoch bei zwischen -20 °C und der Siedetemperatur des Lösungsmittels liegen. Wird eine Temperatur von -20 °C unterschritten, so ist ggf. die Reaktionsgeschwindigkeit der Reduktionsreaktion zu gering.

Das Reduktionsverfahren der Erfindung ist auf ein breites Spektrum von Verbindungen anwendbar. So gehören zu den Aminosäuren oder Aminosäurenderivaten der Formel I mit n = 0 insbesondere α-Aminosäuren oder α-Aminosäurederivate der Formel III 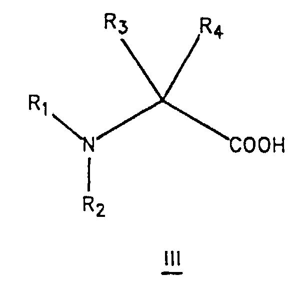 oder mit n = 1 β-Aminosäuren oder deren Derivate der Formel IV 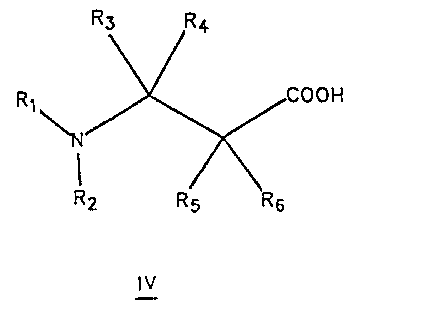 worin jeweils R¹ bis R⁶ die bei Formel I angegebene Bedeutung haben und auch den dort angegebenen Randbedingungen unterliegen, insbesondere auch hinsichtlich eines ggf. vorliegenden Ringes zwischen R¹ oder R² und R³, R⁴, R⁵ oder R⁶.

Besonders vorteilhaft werden in der Erfindung Verbindungen reduziert, in denen ein Ringschluß zwischen R² = Carbonyl und R⁵ = Sauerstoff vorliegt.

In diesen Fällen ist weiterhin bevorzugt R¹ = R⁶ = R⁴ = H und die zu reduzierenden Verbindungen gehorchen der allgemeinen Formel V 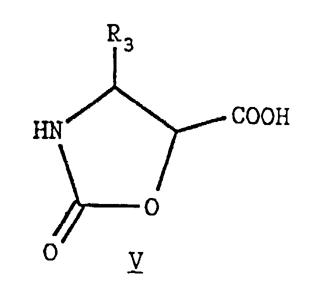 worin R³ die bei Formel I angegebene Bedeutung haben kann.

Besonders vorteilhaft am Verfahren der Erfindung ist die Einsparung von Isolierungsschritten, die Verwendung von billigen Reagentien und Lösungsmitteln sowie der geringe Überschuß an Borhydrid.

Die Aufarbeitung der erfindungsgemäß erhältlichen Aminoalkohole kann nach bekannten Verfahren erfolgen, wobei vorteilhaft zunächst überschüssiges Reduktionsmittel und stabile Bor-Amin/Alkoholat-Komplexe nach Abdestillieren des organischen Lösungsmittel durch vorsichtigen Zusatz von Wasser oder wässriger Säure zerstört werden. Anschließend wird ein ggf. kristallines Reaktionsprodukt abgetrennt oder direkt oder nach Basischstellung der wässrigen Phase das Reaktionsprodukt, ggf. nach weiterer Derivatisierung an einer der freien Funktionalitäten, mit einem geeigneten organischen Lösungsmittel extrahiert und durch Destillation, Kristallisation oder Chromatographie erforderlichenfalls weiter gereinigt.

Das Verfahren wird durch die nachfolgenden Beispiele weiter erläutert:

### Beispiel 1:

### Z-L-Phenylalaninol

9 g (30 mmol) Z-Phenylalanin wurden in 100 ml Ethanol gelöst und nach Zusatz von 0.32 ml Konz. H₂SO₄ (6 mmol) 2 Stunden refluxiert. HPLC zeigte nach dieser Zeit einen Umsatz von ca. 98 % zum Z-Phenylalanin-ethylester. Die Lösung wurde auf Raumtemperatur abgekühlt, mit 0.4 g gemörsertem NaOH (10 mmol) versetzt und zu einer Suspension von 1.57 g (42 mmol) NaBH₄ in 100 ml Ethanol innerhalb von 15 min zugetropft. Nach Beendigung der Reaktion wurde der Alkohol abdestilliert und der Rückstand in 100 ml Wasser aufgenommen. Durch Zusatz von konz. Salzsäure wurde ein pH-Wert von 3 eingestellt. Nach Beendigung der Schaumbildung wurde mit NaOH ein pH-Wert von 12 eingestellt und einmal mit heißem Toluol extrahiert. Die Toluolphase wurde aufkonzentriert und anschließend einige Stunden bei 4 °C belassen. Der ausgefallene Feststoff wurde durch eine Filternutsche abgetrennt, mit Hexan gewaschen und im Vakuum getrocknet.
- Ausbeute:: 6.5 g = 72 % d. Th. (die Wasserphase enthält noch 10 - 15 % d. Th. Produkt, die mit einer weiteren Extraktion mit Toluol isoliert werden können).
- NMR :: entspricht Referenz
- Reinheit (HPLC) :: > 99 %

### Beispiel 2:

### (4S, 5S)-4-Benzyl-5-hydroxymethyl-oxazolidin-2-on

22.1 g (100 mmol) (4S, 5S)-4-Benzyl-oxazolidin-2-on-5-carbonsäure wurden in 120 ml Ethanol gelöst. Es wurden 1.2 ml konz. Schwefelsäure (11 mmol) zugesetzt und 2 Stunden refluxiert. Anschließend wurden 90 ml Ethanol abdestilliert. Diese Lösung wurde nun zu einer Suspension von 5.3 g (140 mmol) NaBH₄ in 40 ml Ethanol innerhalb von 30 min zugetropft. Nach beendeter Zugabe wurde 3 Stunden nachgerührt. Zur farblosen Suspension wurden vorsichtig 100 ml Wasser zugesetzt und mit konz. HCl ein pH-Wert von 7 eingestellt. Nach 2 Stunden Rühren bei Raumtemperatur wurde für weitere 2 Stunden auf 0 °C abgekühlt. Der Feststoff wurde abfiltriert und mit wenig Eiswasser gewaschen. Anschließend wurde im Vakuum bei 45 °C getrocknet.
- Ausbeute:: 18.68 g (90.1 % d. Th.) farbloser Feststoff
- NMR :: entspricht Referenz
- Reinheit (HPLC) :: 99 %

### Beispiel 3:

### Z-L-Threoninol

50.65 g (200 mmol) Z-L-Threonin wurden in 200 ml Ethanol gelöst und nach Zusatz von 2.2 mml konz. H₂SO₄ (40 mmol) 2,5 Stunden refluxiert. Es wurden 100 ml Toluol zugesetzt und 250 ml abdestilliert. Anschließend wurden 200 ml Ethanol zugesetzt, auf Raumtemperatur abgekühlt und in eine Suspension von 12.5 g (330 mmol) NaBH₄ in 200 ml Ethanol innerhalb von 75 min zugetropft. Nach einer Stunde Reaktionszeit wurde mit 100 ml Wasser versetzt und mit konz. HCl auf pH 7 gestellt. Anschließend wurde weitgehend eingedampft und der Rückstand mit 400 ml Wasser aufgenommen. Die wässrige Phase wurde einmal mit 250 ml Ethylacetat und zweimal mit je 100 ml Ethylacetat extrahiert. Nach Abdestillieren des Ethylacetats wurden 47,3 g Öl erhalten, die zu 88 % Produkt enthalten.
- Ausbeute:: 47,3 g Öl (88 %ig)
- NMR :: entspricht Referenz, enthält Ethylacetat
- Reinheit HPLC:: > 98 %

### Beispiel 4:

### L-Valinol

58.6 g (500 mmol) L-Valin wurden in 500 ml Ethanol suspendiert und nach Zusatz von 100 g konz. H₂SO₄ (1 mol) 5 Stunden refluxiert. Es wurden 40 ml Ethanol zugesetzt und auf Raumtemperatur abgekühlt. 120 ml der Lösung (ein Fünftel) wurden nun in eine Suspension von 5.7 g (150 mmol) NaBH₄ in 100 ml Ethanol innerhalb von 75 min zugetropft. Nach 12 Stunden Reaktionszeit wurde das Ethanol im Vakuum abdestilliert, der Rückstand mit 100 ml Wasser versetzt und mit 20 %iger NaOH ein pH-Wert von 12 eingestellt. Die Wasserphase wurde zweimal mit je 150 ml Methylenchlorid extrahiert , die Extrakte über Natriumsulfat getrocknet und im Vakuum weitgehend eingedampft. Man erhielt eine farblose, leicht bewegliche Flüssigkeit.
- Ausbeute:: 11.3 g farbloses Öl
- Titration:: 66.4 %ig = 72.8 % d. Theorie
- Dünnschichtchromatographie:: entspricht Referenz,
einheitlich.

### Beispiel 5:

### (4S, 5S)-4-Benzyl-5-hydroxymethyl-oxazolidinon-2-on

1,1 g (5 mmol) (4S, 5S)-4-Benzyl-oxazolidin-2-on-5-carbonsäure wurden in 15 ml Ethanol gelöst. Es wurden 0,03 ml Schwefelsäure zugesetzt und 4 Stunden refluxiert. Nach dem Abkühlen wurden 0,04 g NaOH zugesetzt und im Vakuum auf 2 g aufkonzentriert. Danach wurden 4 ml Ethanol zugesetzt und in kleinen Portionen bei Raumtemperatur 0,25 g NaBH₄ zugegeben. Nach beendeter Zugabe und weiteren 0,5 Stunden Rühren, war das Edukt bereits vollständig umgesetzt. Es wurden 4 ml Wasser zugesetzt, mit 1,2 ml 10 %iger HCl auf pH 7 neutralisiert und nach Abkühlen auf 0 °C abgekühlt. Der Feststoff wurde über eine Nutsche abgetrennt und aufgrund des kleinen Ansatz mit überproportional viel Wasser gewaschen. Es resultierte eine Isolierausbeute von 0,66 g = 63,7 %, wobei in der Mutterlauge und dem Waschwasser noch ca. 0,2 g = 20 % d. Th. verblieben. Die Diastereomerenreinheit war S,S : R,S = 98,75 : 1.

Weitere Ausführungsformen und Vorteile der Erfindung ergeben sich aus den nachfolgenden Ansprüchen.

## Patentansprüche

1. Verfahren zur Reduktion von Aminosäuren und deren Derivaten der Formel I 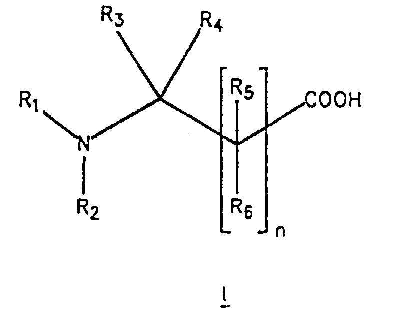 worin
n = 0 oder 1 ist,
R¹, R², R³, R⁴, R⁵ und R⁶ unabhängig voneinander gleich oder verschieden H, Aryl, Alkyl oder Arylalkyl bedeuten, wobei bei den beiden letzteren die Kohlenstoffkette mit Heteroatomen wie N, O oder S oder solche Atome aufweisenden Gruppen substituiert und/oder unterbrochen sein kann,
außerdem
R¹ und R² auch noch unabhängig voneinander gleich oder verschieden Arylalkyloxycarbonyl, Alkyloxycarbonyl oder Oxycarbonyl bedeuten kann,
außerdem
R³, R⁴, R⁵ und R⁶ auch noch unabhängig voneinander gleich oder verschieden Acylaminoalkyl oder Hydroxyl bedeuten können,
und
wobei
R³, R⁴, R⁵ oder R⁶ mit R¹ oder R² einen Ring bilden kann,
zu den entsprechenden Aminoalkoholen der allgemeinen Formel II 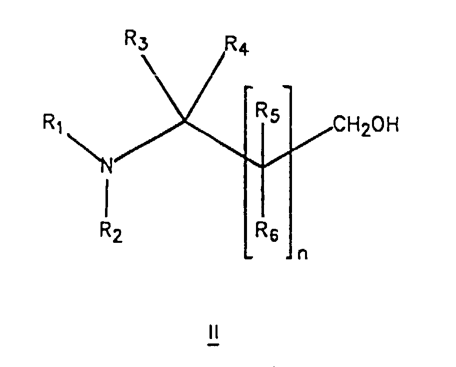 worin R¹ bis R⁶ und n die bei Formel I angegebene Bedeutung aufweisen, bei welchem Verfahren die Verbindungen der Formel I in einem ersten Schritt zunächst in wenigstens einem linearen oder verzweigtkettigen Alkohol mit 1 - 5 C-Atomen unter Zusatz von Säure und unter Erwärmen zu einem Ester umgesetzt werden, wobei eine den Ester enthaltende Reaktionsmischung resultiert und der Ester in einem zweiten Schritt mit Alkali- oder Erdalkaliborhydrid zu Verbindungen der Formel II reduziert wird,
**dadurch gekennzeichnet**,
daß der zweite Schritt ohne Isolierung des Esters aus der Reaktionsmischung ausgeführt wird und daß
Alkali- oder Erdalkaliborhydride ohne Zusatz einer die Reduktionsmittel aktivierenden Substanz zur aus dem ersten Schritt resultierenden Reaktionsmischung verwendet werden.

2. Verfahren nach Anspruch 1,
**dadurch gekennzeichnet**,
daß das Alkali- oder Erdalkaliborhydrid in 1 - <5fachem molaren Überschuß, bevorzugt 1,1 - 3fachem molaren Überschuß, bezogen auf die Verbindung der Formel I, verwendet wird.

3. Verfahren nach einem der Ansprüche 1 bis 2,
**dadurch gekennzeichnet**,
daß im ersten Schritt als Säure HCl, Schwefelsäure oder para-Toluolsulfonsäure eingesetzt wird.

4. Verfahren nach einem der Ansprüche 1 bis 3,
**dadurch gekennzeichnet**,
daß als Alkali- oder Erdalkaliborhydrid Natriumborhydrid, Lithiumborhydrid, Kaliumborhydrid oder Calciumborhydrid verwendet wird.

5. Verfahren nach einem der Ansprüche 1 bis 4,
**dadurch gekennzeichnet**,
daß der Reaktionsmischung nach dem ersten Schritt und vor dem zweiten Schritt Base zugesetzt wird.

6. Verfahren nach einem der vorhergehenden Ansprüche,
**dadurch gekennzeichnet**,
daß die Reduktion mit Borhydrid in einem Temperaturbereich von -20 °C bis Siedetemperatur der Reduktionsmischung durchgeführt wird.

7. Verfahren nach einem der vorhergehenden Ansprüche,
**dadurch gekennzeichnet**,
daß als Verbindung I eine Substanz der Formel V 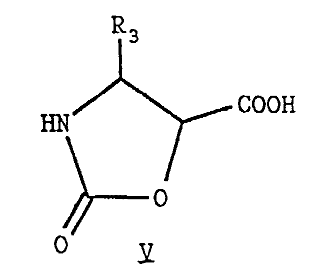 eingesetzt wird, worin R³ die bei Formel I angegebene Bedeutung haben kann.

## Claims

1. Process for the reduction of amino acids and the derivatives thereof of the formula I 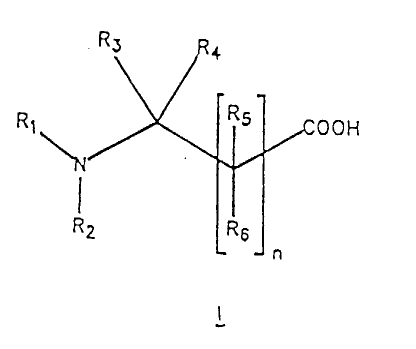 in which
n equals 0 or 1,
R¹, R², R³, R⁴, R⁵, R⁶ are mutually independently identical or different and mean H, aryl, alkyl or arylalkyl, wherein in the latter two cases the carbon chain may be substituted and/or interrupted by heteroatoms such as N, O or S or groups containing such atoms,
moreover
R¹ and R² may also mutually independently be identical or different and mean arylalkyloxycarbonyl, alkyloxycarbonyl or oxycarbonyl,
moreover
R³, R⁴, R⁵ and R⁶ may also mutually independently be identical or different and mean acylaminoalkyl or hydroxyl,
and
wherein
R³, R⁴, R⁵ or R⁶ may form a ring with R¹ or R², to yield the corresponding amino alcohols of the formula II 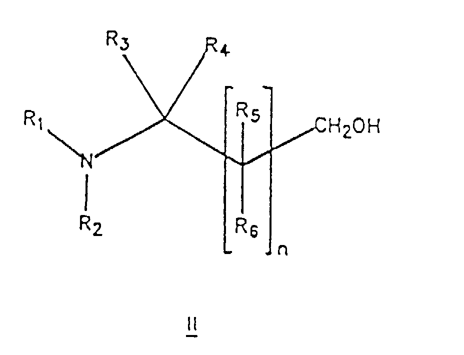 in which R¹ to R⁶ and n have the meaning stated for the formula I, in which process the compounds of the formula I are initially reacted in a first step in at least one linear or branched-chain alcohol having 1-5 C atoms with the addition of acid and with heating to yield an ester, wherein a reaction mixture containing the ester is obtained and the ester is reduced in a second step with alkali metal or alkaline earth metal borohydride to yield compounds of the formula II,
characterised in that
the second step is performed without isolating the ester from the reaction mixture
and that
alkali metal or alkaline earth metal borohydrides are used without the addition of a reducing agent activating substance to the reaction mixture produced in the first step.

2. Process according to claim 1,
characterised in that
the alkali metal or alkaline earth metal borohydride is used in a 1-<5 times molar excess, preferably in a 1.1-3 times molar excess, relative to the compound of the formula I.

3. Process according to one of claims 1 to 2,
characterised in that
the acid used in the first step is HCl, sulphuric acid or para-toluenesulphonic acid.

4. Process according to one of claims 1 to 3,
characterised in that
the alkali metal or alkaline earth metal borohydride used is sodium borohydride, lithium borohydride, potassium borohydride or calcium borohydride.

5. Process according to one of claims 1 to 4,
characterised in that
base is added to the reaction mixture after the first step and before the second step.

6. Process according to one of the preceding claims,
characterised in that
the reduction with borohydride is performed in a temperature range from -20°C to the boiling temperature of the reduction mixture.

7. Process according to one of the preceding claims,
characterised in that
a substance of the formula V 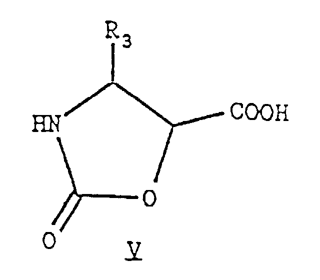 in which R³ has the meaning stated for the formula I, is used as the compound I.

## Revendications

1. Procédé de réduction d'acides aminés et de leurs dérivés de formule I 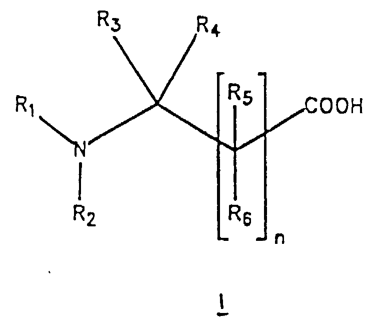 dans laquelle
- n vaut 0 ou 1,
- R¹, R², R³, R⁴, R⁵ et R⁶ représentent indépendamment l'un de l'autre, de manière identique ou différente, H, un aryle, un alkyle ou un arylalkyle, dans ces deux derniers la chaîne carbonée pouvant être substituée par des hétéroatomes, N, O ou S ou des groupes présentant de tels atomes et/ou être interrompue,
- R¹ et R² peuvent encore représenter indépendamment l'un de l'autre, de manière identique ou différente, un arylalkyloxy carbonyle, un alkyloxycarbonyle ou un oxycarbonyle,
- R³, R⁴, R⁵ et R⁶ peuvent également représenter indépendamment l'un de l'autre, de manière identique ou différente, un acylaminoalkyle ou un hydroxyle, et dans laquelle :
- R³, R⁴, R⁵ ou R⁶ peuvent former avec R¹ ou R² un noyau, pour donner les amino-alcools correspondants de formule générale II 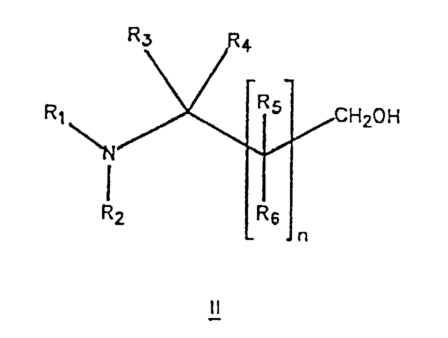
dans laquelle
R¹ à R⁶ et n ont la signification donnée dans la formule I, procédé dans lequel on fait tout d'abord réagir les composés de formule I dans une première étape dans au moins un alcool linéaire ou à chaîne ramifiée ayant de 1 à 5 atomes de carbone avec addition d'un acide et en chauffant pour donner un ester, opération dans laquelle on obtient un mélange réactionnel contenant l'ester et on réduit l'ester dans une seconde étape avec un borohydrure de métal alcalin ou alcalino-terreux afin d'aboutir aux composés de formule II,
caractérisé en ce qu'
- on conduit la seconde étape sans isoler l'ester à partir du mélange réactionnel,
- l'on utilise des borohydrures de métaux alcalins ou alcalino-terreux sans addition d'une substance activant le réducteur, au mélange réactionnel résultant de la première étape.

2. Procédé selon la revendication 1,
caractérisé en ce qu'
on utilise le borohydrure de métal alcalin ou alcalino-terreux en un excès molaire de 1 à moins de 5, de préférence un excès molaire de 1,1 à 3, par rapport au composé de formule I.

3. Procédé selon l'une des revendications 1 à 2,
caractérisé en ce que
dans la première étape on utilise comme acide HCl, l'acide sulfurique ou l'acide para-toluène sulfonique.

4. Procédé selon l'une des revendications 1 à 3,
caractérisé en ce qu'
on utilise comme borohydrure de métal alcalin ou alcalino-terreux le borohydrure de sodium, le borohydrure de lithium, le borohydrure de potassium ou le borohydrure de calcium.

5. Procédé selon l'une des revendications 1 à 4,
caractérisé en ce qu'
on ajoute au mélange réactionnel une base après la première étape et avant la seconde étape.

6. Procédé selon l'une des revendications précédentes,
caractérisé en ce qu'
on conduit la réduction avec du borohydrure dans un intervalle de température de -20°C à la température d'ébullition du mélange réactionnel.

7. Procédé selon l'une des revendications précédentes,
caractérisé en ce qu'
on utilise comme composant I une substance de formule V 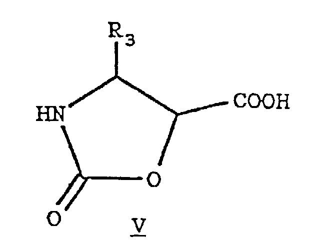 dans laquelle R³ a la signification donnée pour la formule I.
